# EUROPEAN PATENT SPECIFICATION

(11) **EP 2 218 734 B1**
(45) Date of publication and mention of the grant of the patent: **05.06.2013**
(21) Application number: 10163303.0
(22) Date of filing: 11.04.1997
(51) Int. Cl.: C07K 14/605, A61K 38/26, G01N 33/68

(54) **Glucagon-like peptide-2 analogs**
Analoge des Glucagon ähnlichen Peptides-2
Analogues de peptide 2 du type glucagon

(30) Priority: 12.04.1996 US 631273
(43) Date of publication of application: 18.08.2010
(62) Divisional of application: 01129072.3
(73) Proprietor: 1149336 ONTARIO INC., Toronto, Ontario M6B 3G3 (CA); NPS Pharmaceuticals, Inc., Bedminster, NJ 07921 (US)
(72) Inventor: Drucker, Daniel, Toronto Ontario M6B 3G3 (CA); Crivici, Anna, San Diego, Ca 92130 (US); Sumner-Smith, Martin, Bolton Ontario L7E 3V4 (CA)
(74) Representative: Maiwald Patentanwalts GmbH

(56) References cited:
- EP-A- 0 612 531
- WO-A-96/32414

## Description

### FIELD OF THE INVENTION

This invention relates to glucagon-related peptides having intestinal tissue growth promoting properties, and to their use therapeutically to treat various medical conditions resulting from the impaired growth or loss of such tissue.

### BACKGROUND TO THE INVENTION

Expression of the glucagon gene yields a tissue-determined variety of peptide products that are processed from the 160 residue proglucagon product. The organization of these peptides within the proglucagon precursor was elucidated by the molecular cloning of preproglucagon cDNAsfrom the rat, hamster and bovine pancreas. These analyses revealed that preproglucagon contains not only the sequence of glucagon and glicentin, but also two additional glucagon-like peptides (GLP-1 and GLP-2) separated from glucagon and each other by two spacer or intervening peptides (IP-I and IP-II). These peptides are flanked by pairs of basic amino acids, characteristic of classic prohormone cleavage sites, suggesting they might be liberated after posttranslational processing of proglucagon (Drucker, Pancreas, V1990, 5(4): 484).

Analysis of the peptides liberated from proglucagon in the pancreatic islets of Langerhans, for instance, suggests the primary pancreatic peptide liberated is the 29-mer glucagon, whereas glicentin, oxyntomodulin, I P-II and the glucagon-like peptides are more prevalent in the small and large intestines. This demonstration that the glucagon-like peptides are found in the bowel has prompted research into the precise structure and putative function(s) of these newly discovered gut peptides. Most studies have focussed on GLP-1, because several lines of evidence suggested that GLP-1 may be an important new regulatory peptide. Indeed, it has been determined that GLP-1 is one of the most potent known peptidergic stimulus for insulin release, an action mediated in a glucose-dependent manner through interaction with receptors on pancreatic B cells. GLP-1 and its derivatives are in development for use in the treatment of diabetics.

The physiological roles of glicentin and oxyntomodulin, the so-called "enteroglucagons", are also under investigation, particularly with respect to regulation of acid secretion and the growth of intestinal cells. Oxyntomodulin is capable of inhibiting pentagastrin-stimulated gastric acid secretion in a dose-dependent manner. The role of glicentin in mediating the changes of intestinal adaptation and growth of the intestinal mucosa has been investigated, and the intestinotrophic effect of glicentin and its therapeutic use have recently been reported by Matsuno et al in EP 612,531 published August 31, 1994.

In contrast to GLP-1 and other glucagon-related peptides, the physiological role of glucagon-like peptide GLP-2 remains poorly understood despite the isolation and sequencing of various GLP-2 homologues including human, rat, bovine, porcine, guinea pig and hamster. Using GLP-2 antisera raised against synthetic versions of GLP-2, various groups have determined that GLP-2 is present primarily in intestinal rather than pancreatic extracts (see Mojsov et al, J. Biol. Chem., 1986, 261(25): 11880; Orskov et al in Endocrinology, 1986, 119(4):1467 and in Diabetologla, 1987, 30: 874 and in FEBS Letters, 1989, 247(2):193; George et al, FEBS Letters, 1985, 192(2):275). With respect to its biological role, Hoosein et al report (FEBS Letters, 1984, 178(1):83) that GLP-2 neither competes with glucagon for binding to rat liver and brain tissues, nor stimulates adenylate cyclase production in liver plasma membranes, but, enigmatically, can stimulate adenylate cyclase in both rat hyopthalamic and pituitary tissue, at 30-50pM concentrations. An elucidation of the physiological role of GLP-2 would clearly be desirable.

### SUMMARY OF THE INVENTION

There have now been discovered analogs of GLP-2 which promote growth of small bowel tissue. It is accordingly a general object of the present invention to provide such GLP-2 analogs and to provide for their use therapeutically and for related purposes.

In one aspect of the disclosure, the GLP-2-analogs exhibit intestinotrophic activity and conform to the structural Formula 1:
R1-(Y1)m-X1-X2-X3-X4-Ser5-Phe6-Ser7-Asp8-(P1)-Leu14-Asp15-Asn16-Leu17-Ala18-X19-X20-Asp21-Phe22-(P2)-Trp25-Leu26-Ile27-Gln28-Thr29-Lys30-(P3)-(Y2)n-R2.
   wherein
   - X1: is His or Tyr
   - X2: is Ala or an Ala-replacement amino acid conferring on said analog resistance to DPP-IV enzyme;
   - X3: is Pro, HPro, Asp or Glu;
   - X4: is Gly or Ala;
   - P1: is Glu-X10-Asn-Thr-Ile or Tyr-Ser-Lys-Tyr;
   - X10: is Met or an oxidatively stable Met-replacement amino acid;
   - X19: is Ala or Thr;
   - X20: is Arg, Lys, His or Ala;
   - P2: is Ile-Asn, Ile-Ala or Val-Gln;
   - P3: is a covalent bond, or is Ile, Ile-Thr or Ile-Thr-Asn;
   - R1: is H or an N-terminal blocking group;
   - R2: is OH or a C-terminal blocking group;
   - Y1: is one or two basic amino acids selected from the group Arg, Lys, and His;
   - Y2: is one or two basic amino acids selected from the group Arg, Lys, and His; and
m and n, independently, are 0 or 1; and
wherein at least one of X1, X2, X3, X4, P1, X10, X19, X20, P2 and P3 is otherthan a wild type, mammalian GLP-2 residue.

Particularly preferred analogs according to Formula 1 are those which are rendered resistant to cleavage by human DPP-IV enzyme by replacing the Ala at position X2 with an alternative amino acid.

More particularly, and according to one aspect of the disclosure, there are provided analogs of a GLP-2 peptide selected from a mammalian GLP-2 species, the analogs having intestinotrophic activity and incorporating, relative to said mammalian GLP-2 peptide, at least one amino acid substitution at a position which is conserved in mammalian GLP-2's. The GLP-2 analogs incorporate a substitution which is an
1) incorporation at position 2 of a replacement amino acid conferring on said analog resistance to Dipeptidyl Peptidase-IV (hereinafter referred to as DPP-IV).

In another of its aspects, the disclosure provides a pharmaceutical composition comprising a GLP-2 analog of the present invention in a therapeutically effective amount, and preferably in an intestinotrophic amount, and a pharmaceutically acceptable carrier.

In a further aspect, the disclosure provides a method for promoting growth of small bowel tissue in a patient in need thereof, comprising the step of delivering to the patient an intestinotrophic amount of a GLP-2 analog of the present invention.

Besides promoting bowel growth, in another of its aspects the invention provides a method for treating a gastrointestinal disease by administering to a patient suffering from gastrointestinal disease a therapeutically effective amount of a GLP-2 analog of the invention, together with a pharmaceutically acceptable carrier, in order to reduce a pathological effect or symptom of the gastrointestinal disease.

### DETAILED DESCRIPTION OF THE INVENTION

The invention relates to therapeutic and related uses of GLP-2 analogs, particularly for promoting growth of tissue of the small bowel. The effect on growth elicited by the present GLP-2 analogs manifests as an increase in small bowel weight, relative to a mock-treated control. In particular, GLP-2 analogs are considered to have "intestinotrophic" activity if, when assessed in the murine model exemplified herein, the analog mediates an increase in small bowel weight of at least 10% relative to a control animal receiving vehicle alone. Particularly suitable for therapeutic use are those analogs which mediate an increase of at least 20% in small bowel weight; preferred for therapeutic use are those which mediate an increase in small bowel weight of 50% or more. Intestinotrophic activity is noted most significantly in relation to the jejunum, including the distal jejunum and particularly the proximal jejunum, and is also noted in the ileum.

In addition to exhibiting intestinotrophic activity as just defined, the GLP-2 analogs of the present disclosure incorporate an amino acid substitution at one or more sites within a GLP-2 peptide "background", which is a mammalian GLP-2 species per se. Thus, the present peptides incorporate an amino acid substitution in the context of human GLP-2, the sequence of which has been reported by many authors, including Buhl et al, J. Biol. Chem, 1988, 263(18):8621.

In one aspect of the disclosure, the Intestinotrophic analogs of GLP-2 conform to the sequence of Formula 1 as follows:
R1-(Y1)m-X1-X2-X3-X4-Ser5-Phe6-Ser7-Asp8-(P1)-Leu14-Asp15-Asn16-Leu17-Ala18-X19-X20-Asp21-Phe22-(P2)-Trp25-Leu26-Ile27-Gln-28-Thr29-Lys30-(P3)-(Y2)n-R2,
wherein
- X1: is His or Tyr
- X2: is Ala or an Ala-replacement amino acid conferring on said analog resistance to DPP-IV enzyme;
- X3: is Pro, HPro, Asp or Glu;
- X4: is Gly or Ala;
- P1: is Glu-X10-Asn-Thr-Ile or Tyr-Ser-Lys-Tyr;
- X10: is Met or an oxidatively stable Met-replacement amino acid;
- X19: is Ala or Thr;
- X20: is Arg, Lys, His or Ala;
- P2: is Ile-Asn, Ile-Ala or Val-Gln;
- P3: is a covalent bond, or is Ile, Ile-Thr or Ile-Thr-Asn;
- R1: is H or an N-terminal blocking group;
- R2: is OH or a C-terminal blocking group;
- Y1: is one or two basic amino acids selected from the group Arg, Lys, and His;
- Y2: is one or two basic amino acids selected from the group Arg, Lys, and His; and
m and n, independently, are 0 or 1; and
wherein at least one of X1, X2, X3, X4, P1, X10, X19, X20, P2 and P3 is other than a wild type, mammalian GLP-2 residue.

Wild-type mammalian GLP-2 residues which occur at a specific position are determined by aligning the sequences of GLP-2's isolated from different mammalian species and comparing the sequence to the human sequence, reproduced below, for convenience:

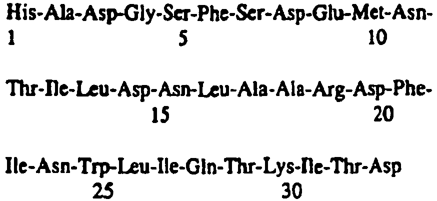

The GLP-2 analog is an analog of full length GLP-2, i.e., GLP-2 (1-33), and P3 is accordingly the sequence Ile-Thr-Asn.

The GLP-2 analog is an analog of human GLP-2 Human GLP-2 is herein referred to interchangably as hGLP-2(1-33).

In particularly preferred embodiments of the disclosure, with respect to the human GLP-2 analogs, the GLP-2 analogs incorporate an amino acid substitution which is an 1) incorporation at X2 of a replacement amino acid which renders said analog resistant to cleavage by DPP-IV enzyme

The DPP-IV-resistant class of GLP-2 analogs possess particularty advantageous properties. As is demonstrated herein, mammalian GLP-2 species have been found to be sensitive to cleavage by DPP-IV enzyme. It has also been found that this sensitivity to DPP-IV is the result of the recognition sequence Ala² Asp³ found In all mammalian forms of GLP-2. There are accordingly provided by the present disclosure a class of GLP-2 analogs which incorporate at X2 a replacement amino acid which confers on the GLP-2 analog relative resistance to DPP-IV mediated cleavage, as determined by any convenient in vitro or in vivo assessment technique that is able to detect the presence of GLP-2 digestion products. A DPP-IV resistant GLP-2 analog is revealed as that GLP-2 analog which is processed or degraded at a rate that is measurably slower than the rate at which human GLP-2 is processed or degraded, under the same conditions.

An assay suitable for assessing DPP-IV sensitivity and resistance is described below in Example 3, in the context of results actually obtained. For purposes of identifying those DPP-IV-resistant X2 analogs that also retain intestinotrophic activity, the X2 analogs showing DPP-IV resistance are screened in the assay of intestinotrophic activity described below in Example 4.

In embodiments of the present disclosure the Ala² replacements is the naturally occurring amino acid Gly There is provided the following Ala²-substituted GLP-2 analog [Gly²]hGLP-2(1-33)

In other embodiments of the disclosure, the GLP-2 analogs incorporate single amino acid substitutions in the context of the mammalian GLP-2 peptide background in which they are introduced.

The present GLP-2 analog can be synthesized using standard techniques of peptide chemistry and can be assessed for intestinotrophic activity, all according to the guidance provided herein. With respect to synthesis, the GLP-2 analog can be prepared by a variety of techniques well known for generating peptide products. Those GLP-2 analogs that incorporate only L-amino acids can be produced in commercial quantities by application of recombinant DNA technology. For this purpose, DNA coding for the GLP-2 analog is incorporated into an expression vector and transformed into a microbial, e.g., yeast, or other cellular host, which is then cultured under conditions appropriate for GLP-2 expression. A variety of gene expression systems have been adapted for this purpose, and typically drive expression of the desired gene from expression regulatory elements used naturally by the chosen host. Because GLP-2 does not require post translational glycosylation for its activity, its production may most conveniently be achieved in bacterial hosts such as E. coli. For such production, DNA coding for the selected GLP-2 peptide may usefully be placed under expression controls of the lac, trp or PL genes of E. coli. As an alternative to expression of DNA coding for the GLP-2 per se, the host can be adapted to express GLP-2 peptide as a fusion protein in which the GLP-2 is linked releasably to a carrier protein that facilitates isolation and stability of the expression product.

In an approach universally applicable to the production of a GLP-2 analog , the well established techniques of automated peptide synthesis are employed, general descriptions of which appear, for example, in J.M. Stewart and J.D. Young, Solid Phase Peptide Synthesis, 2nd Edition, 1984, Pierce Chemical Company, Rockford, Illinois; and in M. Bodanszky and A. Bodanszky, The Practice of Peptide Synthesis, 1984, Springer-Verlag, New York; Applied Biosystems 430A Users Manual, 1987, ABI Inc., Foster City, California. In these techniques, GLP-2 analog is grown from its C-terminal, resin-conjugated residue by the sequential addition of appropriately protected amino acids, using either the Fmoc or tBoc protocols, as described for instance by Orskov et al, 1989, supra.

Once the GLP-2 analog has been synthesized, cleaved from the resin and fully deprotected, the peptide is then purified to ensure the recovery of a single oligopeptide having the selected amino acid sequence. Purification can be achieved using any of the standard approaches, which include reversed-phase highpressure liquid chromatogrephy (RP-HPLC) on alkylated silica columns, e.g., C₄-, C₈-, or C₁₈- silica. Such column fractionation is generally accomplished by running linear gradients, e.g., 10-90%, of increasing % organic solvent, e.g., acetonitrile, in aqueous buffer, usually containing a small amount (e.g., 0.1%) of pairing agent such as TFA or TEA. Alternatively, ion-exchange HPLC can be employed to separate peptide species on the basis of their charge characteristics. Column fractions are collected, and those containing peptide of the desired/required purity are optionally pooled. In one embodiment of the invention, the GLP-2 analog is then treated in the established manner to exchange the cleavage acid (e.g., TFA) with a pharmaceutically acceptable acid, such as acetic, hydrochloric, phosphoric, maleic, tartaric, succinic and the like, to generate a pharmaceutically acceptable acid addition salt of the peptide.

For administration to patients, the GLP-2 analog or its salt is desirably provided in pharmaceutically acceptable form, e.g., as a preparation that is sterile-filtered, e.g., through a 0.22µ filter, and substantially pyrogen-free. Desirably, the GLP-2 analog to be formulated migrates as a single or individualized peak on HPLC, exhibits uniform and authentic amino acid composition and sequence upon analysis thereof, and otherwise meets standards set by the various national authorities which regulate quality of pharmaceutical products.

Fortherapeutic use, the GLP-2 analog is formulated with a carrier that is pharmaceutically acceptable and is appropriate for delivering the peptide by the chosen route of administration. Suitable pharmaceutically acceptable carriers are those used conventionally with peptide-based drugs, such as diluents, excipients and the like. Reference may be made to "Remington's Pharmaceutical Sciences", 17th Ed., Mack Publishing Company, Easton, Penn., 1985, for guidance on drug formulations generally. In one embodiment of the invention, the compounds are formulated for administration by infusion, e.g., when used as liquid nutritional supplements for patients on total parenteral nutrition therapy, or by injection, e.g., sub-cutaneously, intramuscular or intravenously, and are accordingly utilized as aqueous solutions in sterile and pyrogen-free form and optionally buffered to physiologically tolerable pH, e.g., a slightly acidic or physiological pH. Thus, the compounds may be administered in a vehicle such as distilled water or, more desirably, in saline, phosphate buffered saline or 5% dextrose solution. Water solubility of the GLP-2 analog may be enhanced, if desired, by incorporating a solubility enhancer, such as acetic acid.

The aqueous carrier or vehicle can be supplemented for use as injectables with an amount of gelatin that serves to depot the GLP-2 analog at or near the site of injection, for its slow release to the desired site of action. Concentrations of gelatin effective to achieve the depot effect are expected to lie in the range from 10-20%. Alternative gelling agents, such as hyaluronic acid, may also be useful as depoting agents.

The GLP-2 analog of the invention may also be formulated as a slow release implantation device for extended and sustained administration of GLP-2 analog. Examples of such sustained release formulations include composites of blocompatible polymers, such as poly(lactic acid), poly(lactic-co-gycolic acid), methylcellulose, hyaluronic acid, collagen, and the like. The structure, selection and use of degradable polymers in drug delivery vehicles have been reviewed in several publications, including, A. Domb et al., Polymers for Advanced Technologies 3:279-292 (1992). Additional guidance in selecting and using polymers in pharmaceutical formulations can be found in the text by M. Chasin and R. Langer (eds.), "Biodegradable Polymers as Drug Delivery Systems, " Vol. 45 of "Drugs and the Pharmaceutical Science," M. Dekker, New York, 1990. Liposomes may also be used to provide for the sustained release of a GLP-2 analog. Details concerning how to use and make liposomal formulations of drugs of interest can be found in, among other places, U.S. Pat. No 4,944,948; U.S. Pat. No. 5,008,050; U.S. Pat. No. 4,921,706; U.S. Pat. No. 4,927,637; U.S. Pat. No. 4,452,747; U.S. Pat. No. 4,016,100; U.S. Pat. No. 4,311,712; U.S. Pat. No. 4,370,349; U.S. Pat. No. 4,372,949; U.S. Pat. No. 4,529,561; U.S. Pat. No. 5,009,956; U.S. Pat. No. 4,725,442; U.S. Pat. No. 4,737,323; U.S. Pat. No. 4,920,016. Sustained release formulations are of particular interest when it is desirable to provide a high local concentration of a GLP-2 analog.

The GLP-2 analog can be utilized in the form of a sterile-filled vial or ampoule, that contains an intestinotrophic amount of the peptide, in either unit dose or multi-dose amounts. The vial or ampoule may contain the GLP-2 analog and the desired carrier, as an administration-ready formulation. Alteratively, the vial or ampoule may contain the GLP-2 peptide in a form, such as a lyophilized form, suitable for reconstitution in a suitable carrier, such as phosphate-buffered saline.

As an alternative to injectable formulations, the GLP-2 analog may be formulated for administration by other routes. Oral dosage forms, such as tablets, capsules and the like, can be formulated in accordance with standard pharmaceutical practice. According to the present invention, the GLP-2 analog is administered to treat patients that would benefit from growth of small bowel tissue. The effects of GLP-2 analog on this tissue, as evidenced by the results exemplified herein, is dramatic and would clearly benefit those patients suffering from diseases or conditions marked by abnormalities in the small intestinal tract mucosa, which include ulcers and inflammatory disorders; congenital or acquired digestion and absorption disorders including malabsorption syndromes; and diseases and conditions caused by loss of small bowel mucosal function particularly in patients undergoing extended parenteral feeding or who, as a result of surgery, have undergone resection of the small bowel and suffer from short-gut syndrome and cul-de-sac syndrome. Therapeutic treatment with GLP-2 analog is administered so as to reduce or eliminate the disease symptoms and/or improve the nutritional status in these patients associated with their reduced intestinal tract mucosal function. For example, GLP-2 analog is administrated to a patient with an inflammatory bowel condition in an amount sufficient to ameliorate the intestinal discomfort and diarrhea caused by the condition. Additionally, GLP-2 analog may be administered to patients with malabsorption disorders so as to enhance the nutritional absorption and thereby improve the nutritional status of such patients.

In general, patients who would benefit from increased small intestinal mass and consequent increased small bowel mucosal function are candidates for treatment with GLP-2 analog. Particular conditions that may be treated with GLP-2 analog include the various forms of sprue including celiac sprue which results from a toxic reaction to α-gliadin from wheat, and is marked by a tremendous loss of villae of the small bowel; tropical sprue which results from infection and is marked by partial flattening of the villae; hypogammaglobulinemic sprue which is observed commonly in patients with common variable immunodeficiency or hypogammaglobulinemia and is marked by significant decrease in villus height. The therapeutic efficacy of the GLP-2 analog treatment may be monitored by enteric biopsy to examine the villus morphology, by biochemical assessment of nutrient absorption, by patient weight gain, or by amelioration of the symptoms associated with these conditions. Other conditions that may be treated with GLP-2 analog, or for which GLP-2 analog may be useful prophylactically, include radiation enteritis, infectious or post-infectious enteritis, regional enteritis (Crohn's disease), small intestinal damage due to toxic orotherchemotherapeutic agents, and petientswith short bowel syndrome.

The therapeutic dosing and regimen most appropriate for patient treatment will of course vary with the disease or condition to be treated, and according to the patient's weight and other parameters. The results presented hereinbelow demonstrate that a dose of GLP-2 peptide equivalent to about 100µg/kg (or less) administered twice daily over 10 days can generate very significant increases in small bowel mass. It is expected that much smaller doses, e.g., in the µg/kg range, and shorter or longer duration or frequency of treatment, will also produce therapeutically useful results, i.e., a statistically significant increase particularly in small bowel mass. Also, it is anticipated that the therapeutic regimen will include the administration of maintenance doses appropriate for reversing tissue regression that occurs following cessation of initial treatment. The dosage sizes and dosing regimen most appropriate for human use are guided by the results herein presented, and can be confirmed in properly designed clinical trials.

An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Primary among these is the amount of GLP-2 normally circulating in the plasma, which is on the order of 151 pmol/ml in the resting state, rising to 225 pmol/ml after nutrient ingestion for healthy adult humans. Orskow, C. and Helst, J.J., 1987, Scand. J. Clin. Lav. Invest. 47:165. Additional factors include the size of the patient, the age of the patient, the general condition of the patient, the particular disease being treated, the severity of the disease, the presence of other drugs in the patient, the in vivo activity of the GLP-2 analog and the like. The trial dosages would be chosen after consideration of the results of animal studies and the clinical literature. It will be appreciated by the person of ordinary skill in the art that information such as binding constants and Ki derived from in vitro GLP-2 binding competition assays may also be used in calculating dosages, as well as the calculated half-life of the GLP-2 analog in vivo.

Atypical human dose of a GLP-2 peptide would be from about 10 µg/kg bodyweight/day to about 10 mg/kg/day, preferably from about 50 µg/kg/day to about 5 mg/kg/day, and most preferably about 100 µg/kg/day to 1 mg/kg/day. As the GLP-2 analogs of the invention can be up to 10 to even 100 times more potent than GLP-2, a typical dose of such a GLP-2 analog may be lower, for example, from about 100ng/kg body weight/day to 1 mg/kg/day, preferably 1µg/kg/day to 500µg/kg/day, and even more preferably µg/kg/day to 100µg/kg/day.

Similarly, the intestinotrophic GLP-2 analogs of the invention are also useful for both augmenting bowel growth and treating inflammatory disorders in livestock and pets.

### Example 1 - GLP-2 analog synthesis

Solid phase peptide synthesis (SPPS) is carried out manually in a 300 milliliter (ml) vessel on a 3 millimole (mmole) scale using 6 grams (g) of chloromethyl (Merrifield) resin (for C-terminal free acid peptides) with a substitution of 0.5 milliequivalents (meq) per gram. Amino acids are protected at the amino-terminus with the t-butyloxycarbonyl (tBoc) group. The side-chains of trifunctional amino acids are protected with the benzyl (Bz, for serine and threonine), benzyloxymethyl (BOM, for histidine), 2-bromobenzyloxycarbonyl (2-BrZ, for tyrosine), 2-chlorobenzyloxycarbonyl (2-CIZ, for lysine), cyclohexyl (cHex, for aspartic and glutamic acids), and tosyl (Tos, for arginine) groups. The first amino acid is coupled to the chloromethyl resin through esterification of the protected amino acid in the presence of potassium fluoride (KF). C-terminal amide peptides are synthesized on a 4-methylbenzhydrylamine (MBHA) resin on a 3 mmol scale using 6 g of resin with a substitution of 0.5 meq/g. The first amino acid is coupled to the MBHA resin according to the procedure described for peptide elongation.

Amino-group deprotection is carried out using 50% trifluoroacetic acid (TFA) in dichloromethane (CH₂Cl₂), followed by neutralization using two washes of 10% triethylamine (Et₃N) in CH₂Cl₂. Peptide elongation is carried out using N,N-dicyclohexylcarbodiimide/1-hydroxybenzotriazole (DCC/HOBt) activation in CH₂Cl₂/dimethylformamide (DMF). The growing peptide chain is capped after each elongation step with 20% Ac₂O in C₂Cl₂. The peptide-resin is washed after each elongation, capping and deprotection step with isopropanol (iPrOH) and methanol (MeOH). The washes are repeated once. N-terminal acetyl peptides are prepared by acetylation of the terminal amino-group with 20% Ac₂O in CH₂Cl₂ after deprotection and neutralization as described. Resin-bound products are routinely cleaved by a low-high procedure using hydrogen fluoride (HF) containing dimethylsulfide (DMS) and p-cresol as scavengers.

Crude peptides are purified by preparative high pressure liquid chromatography (HPLC) using a Vydac C18, 15-20 µm wide-pore, 2 inch x 12 inch, reverse-phase silica column using gradient elution with 0.1% TFA in water modified with acetonitrile. Elution is monitored at 220 nanometers (nm). Each fraction collected is analyzed for purity by analytical HPLC using a Vydac C18, 5 µm, 4.6 x 254 millimeter (mm), reverse-phase silica column by gradient elution using 0.1% TFA in water modified with acetonitrile, and monitored at 215 nm. Fractions demonstrating greater than 95% purity are combined and lyophilized. Acetate salts of the peptides are prepared from the TFA salts by dissolution of the lyophilized powder in water, with addition of acetonitrile to aid in dissolution where necessary. The solution is passed through a protonated Bio-Rex-70 cation exchange resin. The resin is washed with 5 bed-volumes of water, and the resin-bound peptide is eluted with 50% acetic acid in water. The eluent is diluted with water and tyophilized.

The final lyophilized powder is analyzed for purity by two analytical reverse-phase HPLC methods using a Vydac C18, 5 µm, 4.6 x 254 mm reverse-phase silica column. The two solvent systems used are a gradient of water adjusted to pH 2.25 with triethylamine phosphate, modified with acetonitrile, and a gradient of 0.1% TFA in water, modified with acetonitrile. The column eluent is monitored at 215 nm. The identity of each product is confirmed by amino acid analysis and by electrocopy-mass spectroscopy.

The GLP-2 analogs are next formulated as described below in Example 2. Each of the GLP-2 analogs is fully soluble in water at room temperature unless otherwise noted.

### Example 2 - GLP-2 analog formulation

The GLP-2 analogs were formulated for injection either in phosphate buffered saline or as a gelatin-containing depot formulation. For the PBS-formulated GLP-2 analog preparations, a 10X stock PBS solution was first prepared, using 80g NaCl (BDH ACS 783), 2g KCI (BDH ACS 645), 11.5g Na₂HPO₄ (Anachemia AC-8460), and 2g KH₂PO₄ (Malinckrodt AR7100), which was brought to a total volume of one litre with sterile distilled water. The final working solution was obtained by 10: 1 dilution of the stock solution with sterile distilled water and adjusted to pH 7.3-7.4 if necessary, using sufficient volumes of 10N Na OH. The working solution was then autoclaved for 30 minutes. In the final working PBS solution, concentrations were 137 mM NaCl, 2.7 mM KCI, 4.3 mM Na₂HPO₄.7H2O, and 1.4mM KH₂PO₄.

The GLP-2 analog, as a powdered peptide, is added to the working PBS solution as required to generate formulations having the desired peptide concentrations. For example, to generate a PBS solution of GLP-2 analog at 130 mg/l, 52mg of GLP-2 analog is dissolved in 40ml of PBS to yield a GLP-2 concentration of 130 µg/ml, 0.5 ml is injected twice daily.

To generate the gelatin-based GLP-2 analog formulations, a gelatin solution was first prepared by dissolving 12 grams of gelatin (Sigma, G-8150 Lot #54HO7241 Type A from Porcine skin [9000-70-8] - 300 Bloom) in 100 ml distilled water. The gelatin solution was then autoclaved, warmed at 37°C, and the GLP-2 peptide previously dissolved in phosphate buffered saline as described above was then added to achieve specific, desired peptide concentrations. For instance, to generate a gelatin-based PBS solution of the GLP-2 at a concentration of 130mg/l, 10 ml of a PBS solution prepared with 5.2 mg of GLP-2 was diluted with 30 ml of the 20% working gelatin solution as first described above. The solution was mixed by gentle pipeting, to yield a final solution of 130mg/l GLP-2 in PBS/15% gelatin.

### Example 3 - Assay for Resistance to Dipeptidyl Peptidase IV

The following peptides were tested for resistance to dipeptidyl peptidase IV (DPP-IV): a control peptide, rGLP-2; the [D-Ala²]rGLP-2 analog; and the [Gly²]rGLP-2 analog. To perform the assay, 2.5 microliters (µl) of a solution of human placental DPP-IV (Calbiochem, La Jolla, CA, cat. # 317624) containing 0.125 milliunits (mU) of enzyme in 50% glycerol, 10 mM Tris, pH 7.8, EDTA and 0.02% NaN₃ was added to 50 µl of a solution of the test peptide prepared at a concentration of 0.2 mg/ml in PBS at pH 7.4. The mixture was incubated at 37°C in a circulating water bath for 24 hours. The incubation was quenched by the addition of 50 µl of a solution of diprotin A prepared at a concentration of 4 mg/ml in PBS. Each peptide was tested in duplicate.

Each sample was analyzed by reverse-phase (RP) HPLC as follows: 90 µl of the quenched incubation mixture was injected onto a Rainin Dynamax 300 A, C18, 5 micron, 4.6 x 250 millimeter column. The samples were eluted with 0.1% trifluoroacetic acid (TFA) in water modified with 0.1% acetonitrile using a linear gradient and a flow rate of 1 ml per minute. Sample components were detected at 214 nanometers (nm). The extent of cleavage was measured by relative integration of the peak corresponding to the cleavage product compared to that of the remaining undigested parent peptide. The cleavage product of the control peptide, rGLP-2(1-33), which should be rGLP-2(3-33), was confirmed to have result from cleavage between residues Ala² and Asp³ by comparison of the retention time of this component to that of a synthetic peptide standard, rGLP-2(3-33), and by collection of the product from the HPLC and analysis by mass spectrometry.

After the 24 hour incubation, 22% of the control peptide, rGLP-2, was cleaved by DPP-IV. No cleavage products were detected for the peptides [D-Ala²]rGLP-2 and [Gly²]rGLP-2 after 24 hours.

### Example 4 - GLP-2 analog assessment

Recipients were CD1 mice obtained from Charles River laboratory (Ontario, Canada). The CD1 mice were aged-matched females at time of injection (n=3-4 per group), 6 weeks of age, unless otherwise specified. The animals were allowed a minimum of 24 hours to acclimatize to the laboratory facility before the initiation of each experiment. Animals were identified by ear punch. The mice were not restricted by diet or activity during the experiments. The light/dark cycle was 12 hours, between 6 pm to 6 am. Controls were age- and sex-matched (n=3-4) animals. Mice were injected subcutaneously, twice a day (b.i.d.), with 2.5µg peptide in a total volume of 0.5 cc of PBS and were monitored daily in the laboratory facility. Animals were sacrificed 10 or 14 days after injection, and were fasted at least 20 hours before sacrifice.

The mice were anaesthetised with CO₂ and exsanguinated by cardiac puncture. Blood was collected in 75 µl of TED (Trasysol; EDTA (5000 KIU/ml: 1.2 mg/ml; Diprotin-A), and the blood was centrifuged at 14 k x g for 5 minutes and the plasma was stored at -70 prior to analysis. The small bowel was removed from the peritoneal cavity, from pylorus to cecum, cleaned weighed and measured. For comparative purpose, sections from each animal were obtained from the identical anatomical position. Fragments each measuring 1.5-2.0 cm In length were obtained 8±2 cm, 18±2 cm, 32±2 cm from pylorus for histomorphometry representing proximal jejunum, distal jejunum and distal ileum. Each small bowel fragment was opened longitudinally on its antimesenteric border in a tissue block and then placed on 10% formalin (vol./vol.) overnight, then transferred to 70% ETOH.

Percentage change in small bowel weight was calculated by dividing the mean change in bowel weight of analog treated mice, relative to mice treated with vehicle only, by the mean bowel weight of mice treated with vehicle only, and multiplying this figure by 100.

Results of intestinotrophic activity assessment are shown in Table 1:

**TABLE 1**

| # | GLP-2 analog | % Increase in small bowel weight |
|---|---|---|
| | (1-33 unless noted) | |
| 1 | rGLP-2 | 45 |
| 2 | [Tyr¹]rGLP-2 | 37 |
| 3 | [D-Ala²]rGLP-2 | 86 |
| 4 | [Gly²]rGLP-2 | 70 |
| 5 | [Val²]rGLP-2 | 65 |
| 6 | [Gly²]hGLP-2 | 59 |
| 7 | [Gly²Ala²⁰]hGLP-2 | 46 |
| 8 | (Gly²Ala¹⁰]hGLP-2 | 33 |
| 9 | [Ala¹Gly⁹]hGLP-2 | 23 |
| 10 | [Gly²Ala³]hGLP-2 | 18 |
| 11 | [Gly²Ala⁴]hGLP-2 | 36 |
| 12 | [Glu³]rGLP-2 | 33 |
| 13 | [Ala⁴]rGLP-2 | 30 |
| 14 | [Tyr⁹Ser¹⁰Lys¹¹Tyr¹²(desile¹³)]hGLP-2 | 41 |
| 15 | [Leu¹⁰]rGLP-2 | 26 |
| 16 | [Nleu¹⁰]rGLP-2 | 52 |
| 17 | [Met SO₂¹⁰]rGLP-2 | 8 |
| 18 | [Lys²⁰]rGLP-2 | 62 |
| 19 | [Val²³Gln¹⁴]hGLP-2 | 36 |
| 20 | Amidated C-term | 23 |
| 21 | [Gly²Ala²⁴]hGLP-2 | 67 |
| 22 | [Gly¹Ala⁸]hGLP-2 | 33 |
| 23 | [Gly²Ala¹¹]hGLP-2 | 42 |
| 24 | [Gly¹Ala²¹]hGLP-2 | 35 |
| 25 | [Gyl²Ala⁹]hGLP-2 | 31 |
| 26 | [Gly²Ala¹⁶]hGLP-2 | 36 |
| 27 | [Gly²Ala¹⁷]hGLP-2 | 36 |
| 28 | [Gly²Ala²⁸]hGLP-2 | 31 |
| 29 | [Gly²Ala⁵]hGLP-2 | 38 |
| 30 | [Gly²Ala³¹]hGLP-2 | 28 |
| 31 | [Gly²Ala²⁷]hGLP-2 | 22 |
| 32 | [Gly²Alal²]hGLP-2 | 18 |
| 33 | [Gly²Ala¹³]hGLP-2 | 18 |
| 34 | [Gly²Ala⁷]hGLP-2 | 22 |
| 35 | [Gly²Ala⁶]hGLP-2 | 18 |

It can be readily seen from the above table that analogs of mammalian GLP-2 molecules can have enhanced Intestinotrophic activity. Furthermore, it is clear that depending on the substitution made, various levels of intestinotrophic activity are manifest. For example, [Gly²]rGLP-2 has a greatly enhanced intestinotrphic activity compared to the naturally occuringmolecule; [Gly²]hGLP-2 has a substantially Increased intestinotrophic activity compared with rGLP-2 and [Leu¹⁰] GLP-2 has less than a 50% Increase in intestinotrophic activity.

The following experiments were conducted to confirm that the increase in bowel weight seen in experimental animals treated with DPP-IV resistant analogs of GLP-2, compared with animals treated with wild-type GLP-2, was attributable in part to the DPP-IV resistant nature of the molecules. This experiment took advantage of the availability of a DPP-IV deficient rat strain, Fisher 334 DPP-IV-rats. The effect of rGLP-2 injections on small bowel weight in the Fisher DPP-IV deficient rats was compared to that in normal Sprague-Dawley rats.

In the following experiments the Sprague-Dawley rats were injected s.c. twice daily with rGLP-2 in the Gelatin formulation. The Fisher rats were injected s.c. twice daily with GLP-2 peptides (both rGLP-2, and rGLP-2 analogs) in PBS.

Normal Sprague-Dawley rats treated with rGLP-2, 2.5µg b.i.d. or 25µg b.i.d., showed no % change in small bowel weight compared to control animals given vehicle alone. However, Fisher 334 DPP-IV-rats (DPP-IV deficient animals) demonstrated approximately a 40-50% increase in % change in small bowel weight when treated with 20µg b.i.d. rGLP-2 compared to animals treated with vehicle only. Moreover, DPP-IV deficient animals treated with 20 µg [Gly²]rGLP-2 showed a 50-60% increase in % change in small bowel weight compared with animals given vector alone. Further, Fisher 344 wild-type rats showed 75-85% increase in small bowel weight, over control animals given vehicle alone, when treated with 20µg b.i.d. of [Gly²]rGLP-2.

The above results strongly indicate that GLP-2 is inactivated in vivo In normal rats by cleavage of the two N-terminal residues by a DPP-IV-like enzyme. Furthermore, these results demonstrate that a GLP-2 analog modified so as to be resistant to cleavage by DPP-IV caused a substantial increase in bowel weight in normal rats, presumably as a result of increased GPL-2 half-life in vivo.

As the DPP-IV cleavage site is conserved in all known naturally occurring forms of GLP-2 it seems likely that in mammals DPP-IV cleavage is an important, and probably the primary mechanism whereby GLP-2 is inactivated in vivo. Importantly, GLP-2 analogs resistant to DPP-IV cleavage have enhanced intestinotrophic activity compared to native form.

### Example 5.

Experiments assessing the small bowel inducing activity of various analogs was repeated as described above for Example 4. In these experiments, the small bowel inducing activity of each analog in mice was calculated relative to that of native rat GLP-2(1-33) (expressed as 100% of activity). Resistance of analogs to DPP-IV cleavage was performed as described above in Example 3. Results are tabulated below in Table 2.

**TABLE 2.**

| Identification | Description of Sequence | % Activity Rel. to rGLP-2 | % Cleaved by DPP-IV |
|---|---|---|---|
| | ALE-0109 | | |
| ratGLP-2(1-33) | Native rat sequence | 100% | 58% |
| ratGLP-2(4-33) | N-3; 3 N-termmal res. removed | 6% | |
| Ac-ratGLP-2(1-33) | N-Acetyl | 44% | 0% |
| anglerfish GLP-1 | Native anglerfish GLP-1 | 7% | |
| [Arg-1]ratGLP-2(-1-33) | Arg added to N-terminus | 11% | 0% |
| ratGLP-2(1-30) | C-3; 3 C-terminal res. removed | 23% | |
| ratGLP-2(1-25) | C-8; 8 C-terminal res. removed | 8% | |
| ratGLP-2(1-33)amide | C-terminal amide | 56% | |
| [Arg-2,Arg-1]ratGLP-2(2-33) | Arg-Arg added to N-terminus | 43% | 0% |
| [DAla2]ratGLP-2(1-33) | Ala2->D-Ala2 | 210% | 0% |
| [Gly2]ratGLP-2(1-33) | Ala2->Gly2 | 196% | 0% |
| (Tyr1]ratGLP-2(1-33) | His1->Tyr1 | 81% | 100% |
| [Ala4]ratGLP-2(1-33) | Gly4->Ala4 | 59% | 72% |
| [Glu3]ratGLP-2(1-33) | Asp3->Glu3 | 65% | 50% |
| [Leu10]ratGLP-2(1-33) | Met10->Leu10 | 51% | |
| [Nle10]ratGLP-2(1-33) | Met10->Nle10 | 100% | 100% |
| [Lys20]ratGLP-2(1-33) | Arg20->Lys20 | 120% | |
| [Ser2,Gln3]hGLP-2(1-33) | Ala2->Ser2, Asp3->Glu3 | 11% | 0% |
| [Val23,Gln24]hGLP-2(1-33) | Ile23->Val23, Asn24->Gln24 | 82% | |
| [Val2]GLP-2(1-33) | Ala2->Val2, TFA | 145% | 0% |
| Degu GLP-2 | Native degu sequence | 70% | |
| [Tyr9,Ser10,Lys11,Tyr12, | Glu9->Tyr9, Met10->Ser10, | 94% | |
| des-Ile13]hGLP-2(1-33) | Asn11->Lys11,Th12->Tyr12, and deletion of Il13 | | |
| [Gly2,Ala8]hGLP-2(1-33) | Ala2->Gly2, Asp8->Ala8 | 120% | |
| [Gly2,Nal11][hGLP-2(1-33) | Ala2->Gly2, Asn11->Ala11 | 150% | |
| [Gly2,Ala21]hGLP-2(1-33) | Ala2->Gly2,Asp21->Ala21 | 125% | |
| (Gly2,Ala24)hGLP-2(1-33) | Ala2->Gly2, Asn24->Ala24 | 139% | |
| [Gly2,Ala25]hGLP-2(1-33) | Ala2->Gly2, Trp25->Ala25 | 18% | |
| [Gly2,Ala26]hGLP-2(1-33) | Ala2->Gly2, Leu26->Ala26 | 28% | |
| [Gly2,Ala27]hGLP-2(1-33) | Ala2->Gly2, lie27->Ala27 | 28% 82% | |
| [Gly2,Ala9]hGLP-2(1-33) | Ala2->Gly2, Glu9->Ala9 | 112% | |
| [Gly2,Ala10]hGLP-2(1-33) | Ala2->Gly2, Met10->Ala10 | 82% | |
| [Gly2,Ala12]hGLP-2(1-33) | Ala2->Gly2, Thr12->Ala12 | 67% | |
| [Gly2,Ala13]hGLP-2(1-33) | Ala2->Gly2, Ile13->Ala13 | 67% | |
| [Gly2,Alal4]hGLP-2(1-33) | Ala2->Gly2, Leu14->Ala14 | 30% | 0% |
| [Gly2,Ala16]hGLP-2(1-33) | Ala2->Gly2, Asn16->Ala16 | 130% | |
| [Gly2,Ala17]hGLP-2(1-33) | Ala2->Gly2, Leu17->Ala17 | 130% | |
| [Gly2,Ala20]hGLP-2(1-33) | Ala2->Gly2, Arg20->Ala20 | 105% | |
| [Gly2,Ala23]hGLP-2(1-33) | Ala2->Gly2, Ila23->Ala23 | 24% | |
| [Gly2,Ala28]hGLP-2(1-33) | Ala2->Gly2, Gln8->Ala28 | 130% | |
| [Gly2,Ala30]hGLP-2(1-33) | Ala2->Gly2, Lys30->Ala30 | 24% | |
| [Gly2,Aka31]hGLP-2(1-33) | Ala2->Gly2, He31->Ala31 | 100% | |
| [Gly2]hGLP-2(1-33) | Ala2->Gly2 | 300% | 0% |
| [Alal,Gly2]hGLP-2(1-33) | His1->Ala1, Ala2->Gly2 | 53% | 0% |
| [Gly2,Ala3]hGLP-2(1-33) | Ala2->Gly2, Asp3->Ala3 | 44% | 0% |
| [Gly2,Ala4]hGLP-2(1-33) | Ala2->Gly2, Gly4->Ala4 | 100% | 0% |
| [Gly2,Ala5]hGLP-2(1-33) | Ala²->Gly2, Ser5->Ala5 | 136% | 0% |
| [Gly2,Ala6]hGLP-2(1-33) | Ala2->Gly2, Phe6->Ala6 | 67% | |
| [Gly2,Ala7]hGLP-2(1-33) | Ala2->Gly2, Ser7->Ala7 | 82% | |
| [Pro1]hGLP-2(1-33) | His1->Pro1 | 12% | 0% |
| (Met(O)10]ratGLP-2(1-33) | Met10 sulfoxide | 18% | |
| [Gln20]hGLP-2(1-33) | Arg20->Gln20 | 80% | |
| [Aspl]hGLP-2(1-33) | His1->Asp1 | 60% | 0% |
| [tBuGly2]hGLP-2 | Ala2->t-butyl-Gly2 | 123% | 0% |
| [Tyr34]hGLP-2(1-34) | Addition of C-terminal Tyr | 135% | |
| [Asp2]hGLP-2(1-33) | Ala2->Asp2 | 83% | 0% |
| [Glu2]hGLP-2(1-33) | Ala2->Glu2 | 93% | 0% |
| [Phe2]hGLP-2(1-33) | Ala2->Phe2 | 50% | 0% |
| [His2]hGLP-2(1-33) | Ala2->His2 | 0% | 0% |
| [Ile2]hGLP-2(1-33) | Ala2->Ile2 | 90% | 0% |
| [Lys2]hGLP-2(1-33) | Ala2->Lys2 | 90% | 0% |
| [Met2]hGLP-2(1-33) | Ala2->Met2 | 170% | 0% |
| [Asn2]hGLP-2(1-33) | Ala2->Asn2 | 67% | 0% |
| [Pro2]hGLP-2(1-33) | Ala2->Pro2 | 50% | 16% |
| [Gln2]hGLP-2(1-33) | Ala2->Gln2 | 100% | 0% |
| [Ser2]hGLP-2(1-33) | Ala2->Ser2 | 167% | 0% |
| [Thr2]hGLP-2(1-33) | Ala2->Thr2 | 117% | 0% |
| (Val2]hGLP-2(1-33) | Ala2->Val2 | 180% | 13% |
| [Tyr2]hGLP-2(1-33) | Ala2->Tyr2 | 57% | 0% |
| [D-Ala2]hGLP-2(1-33) | Ala2->D-Ala2 | 130% | 0% |
| hGLP-2(1-33) | Native human sequence | 100% | 60% |
| [desNH2Tyr1]hGLP-2(1-33) | His1->desamino-Tyr1 | 125% | 15% |
| [Thr5]hGLP-2(1-33) | Ser5->Thr5 | 73% | |
| [Ser16,Arg17,Arg18] | Asn16->Ser16, Leu17->Arg17, | 59% | |
| hGLP-2(1-33) | Ala18->Arg18 | | |
| [Asn33]hGLP-2(1-33) | Asp33->Asn33 | 91% | |
| [Pen2]hGLP-2(1-33) | Ala2->L-penicillamine2 | 58% | 20% |
| [bAla2]hGLP-2(1-33) | Ala2->beta-Ala2 | 118% | 0% |
| [aAbu2]hGLP-2(1-33) | Ala2->alpha-aminobutyric acid2 | 84% | 49% |
| [Nva12]hGLP-2(1-33) | Ala2->Nva12 (norvaline) | 111% | 0% |
| [PhGly2]hGLP-2(1-33) | Ala2->L-phenyl-Gly2 | 37% | 0% |
| [Agm34]hGLP-2(1-34) | Addition of C-terminal agmatine | 97% | |
| | (Agm is des-COOH Arg) | | |
| [Arg30]hGLP-2(1-33) | Lys30->Arg30 | 103% | 59% |
| [Pro3]hGLP-2(1-33) | Asp3->Pro3 | 140% | 0% |
| [Ala5, Ala7]hGLP-2(1-33) | Ser5->Ala5, Ser7->Ala7 | 108% | 77% |
| [Tyr1, Gly2]hGLP-2(1-33) | His1->Tyr1, Ala2->Gly2 | 241% | 0% |
| [Glu33]hGLP-2(1-33) | Asp33->Glu33 | 121% | 55% |
| [Phe25]hGLP-2(1-33) | Trp25->Phe25 | 114% | 54% |
| [Tyr25]hGLP-2(1-33) | Trp25->Tyr25 | 64% | 48% |
| [Gly2, Tyr34]hGLP-2(1-34) | Ala2->Gly2 + C-terminal Tyr | 297% | 0% |
| [Aib2]hGLP-2(1-33) | Ala2->aminoisobutyric acid2 | 202% | 0% |
| [Gly2, Arg34)hGLP-2(1-34) | Ala2->Gly2+C-terminal Arg | -250% | 0% |

## Claims

1. [Gly²]hGLP-2(1-33) for use in the treatment of a gastrointestinal disease in a patient or animal.

2. [Gly²]hGLP-2(1-33) for use in the prophylaxis or treatment of conditions selected from radiation enteritis, infectious or post-infectious enteritis, regional enteritis (Crohn's disease), small intestinal damage due to toxic or other chemotherapeutic agents, and short bowel syndrome.

3. [Gly²]hGLP-2(1-33) for use according to claim 1, wherein the gastrointestinal disease is selected from the group consisting of inflammatory bowel condition, celiac sprue, tropical sprue and hypogammaglobulinemic sprue.

4. [Gly²]hGLP-2(1-33) for use according to any of claims 1 to 3, wherein [Gly²]HGLP-2(1-33) is administered to a human in a dose of 100 ng/kg body weight/day to 1 mg/kg body weight/day.

5. [Gly²]hGLP-2(1-33) for use according to any of claims 1 to 3, wherein [Gly²]HGLP-2(1-33) is administered to a human in a dose of 1 µg/kg body weight/day to 500 µg/kg body weight/day.

6. [Gly²]hGLP-2(1-33) for use according to any of claims 1 to 3, wherein [Gly²]HGLP-2(1-33) is administered to a human in a dose of 1 µg/kg body weight/day to 100 µg/kg body weight/day.

7. [Gly²]hGLP-2(1-33) for use according to any of claims 1 to 6, wherein [Gly²]hGLP-2(1-33) is provided in a pharmaceutically acceptable form.

8. [Gly²]hGLP-2(1-33) for use according to any of claims 1 to 7, wherein [Gly²]HGLP-2(1-33) is formulated with a pharmaceutically acceptable carrier.

9. [Gly²]hGLP-2(1-33) for use according to any of claims 1 to 8, wherein [Gly²]hGLP-2(1-33) is formulated for administration by infusion or injection.

10. [Gly²]hGLP-2(1-33) for use according to claim 9, wherein injection is subcutaneously, intramuscularly or intravenously.

11. [Gly²]hGLP-2(1-33) for use according to claim 9 or 10, wherein [Gly²]hGLP-2(1-33) is formulated as an aqueous solution that is sterile and pyrogen-free.

12. [Gly²]hGLP-2(1-33) for use according to claim 11, wherein the aqueous solution is buffered to a physiologically tolerable pH.

13. [Gly²]hGLP-2(1-33) for use according to any of claims 1 to 12, wherein an intestinotrophic amount of [Gly²]hGLP-2(1-33) is utilized in the form of a sterile-filled vial or ampoule and the vial or ampoule contains [Gly²]hGLP-2(1-33) in a lyophilized form suitable for reconstitution in an suitable carrier.

14. Use of [Gly²]hGLP-2(1-33) in the manufacture of a medicament for the treatment of a gastrointestinal disease in a patient or animal.

## Patentansprüche

1. [Gly²]hGLP-2(1-33) zur Verwendung in der Behandlung einer gastrointestinalen Krankheit in einem Patienten oder Tier.

2. [Gly²]hGLP-2(1-33) zur Verwendung in der Prophylaxe oder Behandlung von Krankheiten ausgewählt aus Strahlenenteritis, infektiöser oder nachinfektiöser Enteritis, lokaler Enteritis (Morbus Crohn), kleinen Darmverletzungen aufgrund toxischer oder chemotherapeutischer Agentien und Kurzdarmsyndrom.

3. [Gly²]hGLP-2(1-33) zur Verwendung gemäß Anspruch 1, wobei die gastrointestinale Krankheit ausgewählt ist aus der Gruppe bestehend aus entzündlicher Darmkrankheit, Zöliakie (celiac sprue), tropischer Sprue und hypogammaglobulinämischer Sprue.

4. [Gly²]hGLP-2(1-33) zur Verwendung gemäß einem der Ansprüche 1 bis 3, wobei [Gly²]hGLP-2(1-33) einem Menschen in einer Dosis von 100 ng/kg Körpergewicht/Tag bis 1 mg/kg Körpergewicht/Tag verabreicht wird.

5. [Gly²]hGLP-2(1-33) zur Verwendung gemäß einem der Ansprüche 1 bis 3, wobei [Gly²]hGLP-2(1-33) einem Menschen in einer Dosis von 1 µg/kg Körpergewicht/Tag bis 500 µg/kg Körpergewicht/Tag verabreicht wird.

6. [Gly²]hGLP-2(1-33) zur Verwendung gemäß einem der Ansprüche 1 bis 3, wobei [Gly²]hGLP-2(1-33) einem Menschen in einer Dosis von 1 µg/kg Körpergewicht/Tag bis 100 µg/kg Körpergewicht/Tag verabreicht wird.

7. [Gly²]hGLP-2(1-33) zur Verwendung gemäß einem der Ansprüche 1 bis 6, wobei [Gly²]hGLP-2(1-33) in einer pharmazeutisch annehmbaren Form zur Verfügung gestellt wird.

8. [Gly²]hGLP-2(1-33) zur Verwendung gemäß einem der Ansprüche 1 bis 7, wobei [Gly²]hGLP-2(1-33) mit einem pharmazeutisch akzeptablen Träger formuliert wird.

9. [Gly²]hGLP-2(1-33) zur Verwendung gemäß einem der Ansprüche 1 bis 8, wobei [Gly²]hGLP-2(1-33) zur Verabreichung durch Infusion oder Injektion formuliert wird.

10. [Gly²]hGLP-2(1-33) zur Verwendung gemäß Anspruch 9, wobei die Injektion subkutan, intramuskulär oder intravenös ist.

11. [Gly²]hGLP-2(1-33) zur Verwendung gemäß Anspruch 9 oder 10, wobei [Gly²]hGLP-2(1-33) als eine wässrige Lösung formuliert wird, die steril und pyrogenfrei ist.

12. [Gly²]hGLP-2(1-33) zur Verwendung gemäß Anspruch 11, wobei die wässrige Lösung auf einen physiologisch tolerierbaren pH gepuffert ist.

13. [Gly²]hGLP-2(1-33) zur Verwendung gemäß einem der Ansprüche 1 bis 12, wobei eine intestinothrophe Menge von [Gly²]hGLP-2(1-33) in Form eines steril gefüllten Fläschchens oder einer steril gefüllten Ampulle verwendet wird und das Fläschchen oder die Ampulle [Gly²]hGLP-2(1-33) in einer lyophilisierten Form geeignet für die Rekonstitution in einem geeigneten Träger enthält.

14. Verwendung von [Gly²]hGLP-2(1-33) zur Herstellung eines Medikaments zur Behandlung einer gastrointestinalen Krankheit in einem Patienten oder Tier.

## Revendications

1. [Gly²]hGLP-2(1-33) destiné à être utilisé dans le traitement d'une maladie gastro-intestinale chez un patient ou un animal.

2. [Gly²]hGLP-2(1-33) destiné à être utilisé dans la prophylaxie ou le traitement d'états sélectionnés parmi l'entérite radio-induite, l'entérite infectieuse ou post-infectieuse, l'entérite régionale (maladie de Crohn), les lésions de l'intestin grêle dues à des agents toxiques ou à d'autres agents chimiothérapeutiques, et le syndrome de l'intestin court.

3. [Gly²]hGLP-2(1-33) destiné à être utilisé selon la revendication 1, où la maladie gastro-intestinale est sélectionnée dans le groupe constitué par l'état inflammatoire de l'intestin, la sprue coeliaque, la sprue tropicale et la sprue hypogammaglobulinémique.

4. [Gly²]hGLP-2(1-33) destiné à être utilisé selon l'une quelconque des revendications 1 à 3, où [Gly²]hGLP-2(1-33) est administré à un humain dans une dose de 100 ng/kg de poids corporel/jour à 1 mg/kg de poids corporel/jour.

5. [Gly²]hGLP-2(1-33) destiné à être utilisé selon l'une quelconque des revendications 1 à 3, où [Gly²]hGLP-2(1-33) est administré à un humain dans une dose de 1 µg/kg de poids corporel/jour à 500 µg/kg de poids corporel/jour.

6. [Gly²]hGLP-2(1-33) destiné à être utilisé selon l'une quelconque des revendications 1 à 3, où [Gly²]hGLP-2(1-33) est administré à un humain dans une dose de 1 µg/kg de poids corporel/jour à 100 µg/kg de poids corporel/jour.

7. [Gly²]hGLP-2(1-33) destiné à être utilisé selon l'une quelconque des revendications 1 à 6, où [Gly²]hGLP-2(1-33) est délivré sous une forme pharmaceutiquement acceptable.

8. [Gly²]hGLP-2(1-33) destiné à être utilisé selon l'une quelconque des revendications 1 à 7, où [Gly²]hGLP-2(1-33) est formulé avec un support pharmaceutiquement acceptable.

9. [Gly²]hGLP-2(1-33) destiné à être utilisé selon l'une quelconque des revendications 1 à 8, où [Gly²]hGLP-2(1-33) est formulé pour une administration par perfusion ou injection.

10. [Gly²]hGLP-2(1-33) destiné à être utilisé selon la revendication 9, où l'injection est sous-cutanée, intramusculaire ou intraveineuse.

11. [Gly²]hGLP-2(1-33) destiné à être utilisé selon la revendication 9 ou 10, où [Gly²]hGLP-2(1-33) est formulé comme solution aqueuse stérile et apyrogène.

12. [Gly²]hGLP-2(1-33) destiné à être utilisé selon la revendication 11, où la solution aqueuse est neutralisée à un pH physiologiquement tolérable.

13. [Gly²]hGLP-2(1-33) destiné à être utilisé selon l'une quelconque des revendications 1 à 12, où une quantité intestinotrophique de [Gly²]hGLP-2(1-33) est utilisée sous la forme d'une fiole ou d'une ampoule remplie de façon stérile et la fiole ou l'ampoule contient [Gly²]hGLP-2(1-33) sous une forme lyophilisée apte à être reconstituée dans un support approprié.

14. Utilisation de [Gly²]hGLP-2(1-33) dans la fabrication d'un médicament pour le traitement d'une maladie gastrointestinale chez un patient ou un animal.


### SEQUENCE LISTING

<110> Ontario Inc.
<120> Glucagon-Like Peptide-2 Analogs
<130> O 7758 / MH
<160> 2
<170> Pat ent I n ver si on 3. 3
<210> 1
   <211> 37
   <212> PRT
   <213> Unknown
<220>
   <223> PEPTIDE
<220>
   <221> misc_feature
   <222> 1
   <223> / note= " A basic amino acid selected from the group Arg, Lys, and His. Xaa may or may not be present in the sequence"
<220>
   <221> m sc_f eat ur e
   <222> 2
   <223> / not e= " A basic amino acid selected from the group Arg, Lys, and His. Xaa may or may not be present in the sequence"
<220>
   <221> var i ant
   <222> 3
   <223> / replace=" Tyr"
<220>
   <221> misc_feature
   <222> 4
   <223> / not e= " Al a or an Al a- replacement amino acid conferring on said analog resistance to DPP-IV enzyme"
<220>
   <221> variant
   <222> 5
   <223> / replace=" HPr o" / replace=" Asp" / replace=" Glu"
<220>
   <221> variant
   <222> 6
   <223> / replace=" Al a"
<220>
   <221> var i ant
   <222> 11
   <223> / replace=" Tyr "
<220>
   <221> misc_feature
   <222> 12
   <223> / note= "Ser if position 11 is Tyr, ot her wi se Met or an oxidatively stable Met replacement amino acid"
<220>
   <221> misc_feature
   <222> 13
   <223> / not e= " Lys if position 11 is Tyr, or Asn"
<220>
   <221> misc_feature
   <222> 14
   <223> / not e= "Tyr if position 11 is Tyr, or Thr
<220>
   <221> misc_feature
   <222> 15
   <223> / note= "Ile, or Xaa is not pr esent if position 11 is Tyr"
<220>
   <221> var i ant
   <222> 21
   <223> / replace=" Thr"
<220>
   <221> var i ant
   <222> 22
   <223> / replace=" Lys" / replace=" His " / replace=" Al a"
<220>
   <221> var i ant
   <222> 25
   <223> / repl ace=" Val "
<220>
   <221> misc_feature
   <222> 26
   <223> / not e= "Xaa is Asn or Al a if position 25 is Ile. Xaa is Gl n if position 25 is Val"
<220>
   <221> misc_feature
   <222> 33
   <223> / not e= "Ile or a covalent bond"
<220>
   <221> misc_feature
   <222> 34
   <223> / not e= "Thr if position 33 is Ile, or a coval ent bond"
<220>
   <221> mi sc_f eat ur e
   <222> 35
   <223> / not e= "Asn i f position 34 is Thr, or a covalent bond"
<220>
   <221> m sc_featur e
   <222> 36
   <223> / not e= " A basic amino acid selected from the gr oup Ar g, Lys, and His. Xaa may or may not be present in the sequence"
<220>
   <221> m sc_f eat ur e
   <222> 37
   <223> / not e= " A basic amino acid sel ect ed from the gr oup Ar g, Lys, and His. Xaa may or may not be present in the sequence"
<220>
   <221> misc_feature
   <222> 3, 5, 6, 11, 21, 22, 25
   <223> / note= " Residues given in the sequence have no pr ef er ence with respect to those in the annot at i ons f or sai d positions"
<400> 1
   Xaa Xaa His Xaa Pro Gly Ser Phe Ser Asp Glu Xaa Xaa Xaa Xaa Leu
   1 5 10 15
   Asp Asn Leu Ala Ala Arg Asp Phe Ile Xaa Trp Leu Ile Gln Thr Lys
   20 25 30
   Xaa Xaa Xaa Xaa Xaa
   35
<210> 2
   <211> 33
   <212> PRT
   <213> Unknown
<400> 2
   His Ala Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp